# EUROPEAN PATENT APPLICATION

(11) **EP 4 671 352 A1**
(43) Date of publication of application: **31.12.2025**
(21) Application number: 24184353.1
(22) Date of filing: 25.06.2024
(51) Int. Cl.: C12M 1/12

(54) **TUBE REACTOR MODULE, USE OF TUBE REACTOR MODULE, AND INCUBATION DEVICE**

(71) Applicant: Sartorius Stedim Biotech GmbH, 37079 Göttingen (DE)
(72) Inventor: DEUSE, Mario, 37079 Göttingen (DE); KRUMBEIN, Thomas, 37079 Göttingen (DE); WEISSHAAR, Stefan, 37079 Göttingen (DE); WORTMEYER, Johannes, 37079 Göttingen (DE); GITT, Silas, 37079 Göttingen (DE)
(74) Representative: Müller-Boré & Partner Patentanwälte PartG mbB

(57) **Abstract**

The application relates to a tube reactor module for controlling a reaction time of a process fluid in dynamic operation, in particular during viral inactivation in the process fluid, comprising: an inlet for receiving the process fluid; an outlet for discharging the process fluid; a tubular flow path comprising a serpentine pattern for guiding the process fluid from the inlet to the outlet, the tubular flow path comprising a sequence of fluidly connected alternatingly bent curve sections, wherein each curve section is shaped such that a flow direction of the process fluid changes by at least approximately 90°, in particular by between approximately 135° and 225°, and wherein the tubular flow path comprises a first cross-section perpendicular to the flow direction of the process fluid at respective apexes of the curve sections and a second cross-section perpendicular to the flow direction of the process fluid at respective end portions of the curve sections, wherein the first cross-section is different from the second cross-section.

## Description

The present disclosure relates to a tube reactor module, a tube reactor system comprising two or more tube reactor modules and use of a tube reactor module for viral inactivation in a process fluid.

Process devices such as incubators comprise reactors for holding and piping a process liquid for a specific process time. For ensuring a high process quality within a (predetermined processing time, precise information of the process devices and process parameters are required.

However, measurement of a device volume of the reactor is difficult and often imprecise. Furthermore, reactors often pipe the process liquid in an irregular manner so that a residence time distribution of the process liquid in the reactor is high. This leads to an insufficient processing quality of the process liquid and/or prolongs the required processing time of the process liquid to ensure sufficient processing progress. However, the processing time of the process liquid should be as short as possible in order to avoid or reduce degradation of the process liquid due to the environment present during processing.

As an example, for viral inactivation, the process liquid is required to have a processing time of at least 30 minutes so that sufficient inactivation is ensured. However, it is desirable to keep the exceedance of said 30 minutes as low as possible so that the substances in the process fluid are not harmed by the high or low pH levels used for viral inactivation. An effective mixing at a low residence time distribution is paramount for high quality and process efficiency.

It is therefore an object of the invention to provide a reactor for processing a process fluid offering improved control of the process parameters, in particular providing effective mixing of the process fluid at a low residence time distribution of the process fluid.

### Summary

One aspect of the invention relates to a tube reactor module for controlling a reaction time of a process fluid in dynamic, in particular continuous, operation, in particular during viral inactivation in the process fluid, comprising: an inlet for receiving the process fluid, an outlet for discharging the process fluid; a tubular flow path comprising a serpentine pattern for guiding the process fluid from the inlet to the outlet, the tubular flow path comprising a sequence of fluidly connected alternatingly bent curve sections, wherein each curve section is shaped such that a flow direction of the process fluid changes by at least approximately 90°, in particular by between approximately 135° and 225°, and wherein the tubular flow path comprises a first cross-section perpendicular to the flow direction of the process fluid at respective apexes of the curve sections and a second cross-section perpendicular to the flow direction of the process fluid at respective end portions of the curve sections, wherein the first cross-section is different from the second cross-section.

A corresponding tube reactor module provides the advantage of sufficient mixing of the process fluid and at the same time offering improved control of a residence time of the process fluid within the tubular flow path. In particular, a corresponding tube reactor module may offer improved homogenization of residence time and/or reduced residence time distribution of the process fluid. Thus, the tube reactor module described herein is particularly advantageous for viral inactivation or similar applications, where an efficient processing is desired but a minimum residence time should not fall below a certain limit.

The tube reactor module provides effective yet gentle mixing due to change of flow direction of the process fluid by a curve section by more than 90°, because a respective change of flow direction causes the occurrence of whirls and/or vortices, which are particularly advantageous for effective mixing.

In general, the larger the change in flow direction, the more and/or larger and/or stronger are the whirls and/or vortices, which improves the mixing result.

In particular, the larger the change in flow direction, the more Dean vortices may occur and/or be caused, which enhances mixing of the process fluid. At the same time, a larger change in flow direction does not influence the Dean number of the tubular flow path.

Enhanced mixing narrows and/or reduces the residence time distribution of the process fluid.

In the tube reactor module, two sequential curve sections may be bent in alternate directions, in particular in substantially opposite directions. Alternatively or in addition, the curve sections may be formed such that the flow direction of the process fluid changes by approximately 180°.

This is particularly advantageous because a substantially planar tubular flow path with a compact volume may be obtained. Also, a corresponding or similar tubular flow path may be manufactured efficiently and/or with low material use.

A plurality of curve sections may be substantially identical and/or substantially uniform. Preferably, the majority of curve sections are substantially identical and/or substantially uniform. This improves uniform mixing of the process fluid along the extent of the tubular flow path.

Advantageously, the tubular flow path comprises a plurality of substantially identical sections in a repeating manner.

Optionally, the shape of the first cross-section and the second cross-section of the curve sections may differ, which increases mixing results due to changing interference of the process fluid with the inner wall of the tubular flow path and/or due to flow velocity variations of the process fluid.

For example, the first cross-section at an apex of the curve sections may be substantially oval and/or elliptical and the second cross-section may be substantially circular. The oval and/or elliptical shape of the first cross-section at an apex of the curve sections may have a height that is greater than the diameter of the second cross-section. Alternatively, the oval and/or elliptical shape of the first cross-section at an apex of the curve sections may have a height that is smaller than the diameter of the second cross-section.

It is preferred that the oval and/or elliptical shape of the first cross-section at an apex of the curve sections is larger in a vertical direction if the changing flow direction of the process fluid remains substantially in a horizontal plane. This provides a more even velocity distribution of the components of the process fluid during passing through the curve section due to a reduced tubular flow path length difference between an inner wall and an outer wall of the curve section.

Alternatively, the first and second cross-sections may comprise the same shape but the orientation of the shape may differ. For example, the shape of the first cross-section may be oval with a height taller than its width and the second cross-section may also be oval but with a width wider than its height or vice versa.

However, the shape of the first cross-section and/or the second cross-section and/or further cross-sections in the curve sections and/or the tubular flow path may comprise various shapes. The shapes may be one or more of: square, rectangle, pentagon, hexagon, heptagon, octagon, nonagon, decagon, triangle, diamond/rhombus, crescent, trapezium, trefoil, rectangle, parallelogram and/or semicircle. Rounded corners are advantageous in order to improve the flow of the process fluid and reduce shear forces.

Alternatively or in addition, the size and/or diameter and/or cross-sectional area of the first cross-section and the second cross-section may differ. Similarly to what is stated above, a difference in cross-sections causes turbulences due to velocity differences in the process fluid and improves mixing of the process fluid.

Alternatively, the size and/or diameter and/or cross-sectional area of the first cross-section and the second cross-section may be substantially identical and/or not differ by more than 10 %. This way, the flow velocity may remain substantially constant over the extent of the curve section, which provides a more reliable and/or predictable control of the reaction time of the process fluid.

The first cross-section and/or the second cross-section may comprise a cross-sectional area of between approximately 0.19 mm² to approximately 8000 mm², in particular between approximately 1 mm² and approximately 1000 mm², further particular between approximately 4 mm² and approximately 200 mm², further particular between approximately 7 mm² and approximately 64 mm².

Accordingly, a diameter and/or width and/or height of the first cross-section and/or the second cross-section may be between approximately 0.5 mm and approximately 100 mm, in particular between approximately 3 mm and approximately 10 mm.

Cross section and/or width and/or diameter of the tubular flow path may influence the flow velocity of the fluid and therefore the dean number and the pressure drop between inlet and outlet.

For example, design considerations of the tubular flow path for a preset volume stream (l/h) advantageously includes selecting a cross-section, which results in a flow velocity of the fluid such that the Dean number is greater than approximately 100.

However, the influence of the Dean number on the residence time distribution is not constant. Thus, at a certain point the residence time distribution may not be significantly further influenced while at the same time, the pressure drop increases significantly with further reduction of cross-sections.

Consequently, the cross section may be selected as trade-off between sufficient residence time distribution and acceptable pressure drop.

A particular example of the tubular flow path may comprise curve sections having an oval and/or elliptical first cross-section with a height of approximately 10,2 mm and a width of approximately 3,9 mm, which results in an average Dean number of 89. The second cross-section of the curve sections may comprise a round cross-section with a diameter of approximately 6 mm, which results in a Dean number of 89. In said example, the cross-sectional areas at the first cross-section and the second cross-section are substantially identical, which results in substantially identical Dean numbers.

If the cross-sectional area at the first cross-section is larger than the cross-sectional area at the second cross-section, the pressure drop at the second cross-section is larger and vice versa.

A ratio of the cross-sectional area of the first cross-section to the cross-sectional area of the second cross-section may be between 0.01 to 100, in particular between 0.1 to 10. Differing cross-sectional areas provide for flow velocity differences between different portions of the tubular flow path, which may improve mixing results.

The ratio may be designed in consideration of the residence time distribution and the pressure drop.

A ratio of the cross-sectional area of the first cross-section to the cross-sectional area of the second cross-section may be approximately 1.

Preferably, the curve sections comprise a substantially smooth cross-section transition between the first cross-section and the second cross-section and vice versa. In general, it is preferred that the tubular flow path comprises a substantially even and/or gradient transition between differing cross-sections and/or the inner wall of the tubular flow path is substantially free from perceptible steps. This way, undesired flow disturbances and/or shear forces may be avoided or at least reduced.

The tubular flow path length may be between approximately 0.1 m and approximately 1000 m, in particular between approximately 10 m and approximately 100 m.

The tubular flow path may comprise between 2 and 10.000 curve sections, in particular between 200 and 1100 curve sections.

The tubular flow path may comprise more than 2 and/or up to 10.000, in particular 10 to 500, in particular 30 to 50, curve sections per meter of a tubular flow path length.

The tubular flow path length and the number of curve sections of a tube reactor module may be determined based on the desired residence time of the process fluid within the tubular flow path under consideration of the flow rate of the process fluid and based on the desired mixing result of the process fluid at the end of the tubular flow path.

A longer tubular flow path length results in a longer residence time of the fluid compared to a shorter tubular flow path length at the same fluid flow rate. A larger number of curve sections result in an improved mixing result compared to a smaller number of curve sections of the same configuration.

The tubular flow path may comprise between 2 and 10.000 curve sections per 5 seconds residence time of the process fluid. According to a particular example, the tubular flow path may comprise 960 curve sections per 30 minutes residence time of the process fluid within the tubular flow path. This provides a useful compromise between mixing result and residence time, in particular advantageous for virus inactivation.

A radius of the curve sections may be between approximately 0.7 mm and approximately 250 mm, in particular between approximately 2 mm and approximately 50 mm, further particular between approximately 5 mm and approximately 20 mm.

The radius of the curve sections may correspond to a radius of a part of a circle, which is defined by midpoints and/or central points along the tubular flow path in the curve section. The radius of the curve sections may alternatively correspond to a radius of the inner wall or of the outer wall of the tubular flow path in the curve section. The smaller the radius, the better the mixing result.

However, the smaller the radius, the larger the pressure drop caused by the curve section and/or the larger the velocity drop of the process fluid.

It is advantageous to obtain a rather small pressure drop so that a constant flow of the process fluid may be sustained over the entire length of the tubular flow path yet a sufficiently small radius to obtain sufficient mixing.

A reduced pressure drop between inlet and outlet is advantageous, in particular in terms of sustainability as it requires lower energy consumption, in particular for pumping device(s) for pumping the process fluid through the tubular flow path, and/or lower mechanical stability of the tubular flow path, so that for example less and/or lighter material may be used.

The radius of the curve sections should be determined under consideration of, for example, a total pressure difference between the inlet and the outlet of the tube reactor module. A pressure applied at the inlet may be between approximately 0.1 bar and approximately 10 bar, for example approximately 3 bar. The pressure drop caused by the tubular flow path, in particular by the curve sections, is preferably smaller than the pressure applied at the inlet so that a constant flow of the process fluid may be obtained.

A curve distance measured between respective apexes of sequential curve sections may be between approximately 5 mm and approximately 100 mm, in particular between approximately 10 mm and approximately 30 mm.

The curve distance may correspond to the distance between inner side, mid point or outer side of the apexes of respective curve sections. The curve distance may be measured in a direction substantially perpendicular to an imaginary line connecting a plurality of adjacent but not sequential curve sections of the tubular flow path.

The tubular flow path may comprise between 100 and 4000 curve sections per 0.5 ml volume, in particular between 500 and 1500 curve sections per 0.5 ml volume. Alternatively or in addition, the tubular flow path may comprise between 2 and 10.000 curve sections per between approximately 0.1 ml and 1 l tubular flow path volume, for example 960 curve sections per 500 ml volume and/or 320 curve sections per 166 ml volume.

A ratio of a cross-section diameter of the first cross-section and/or the second cross-section and a tubular flow path length is between approximately 0.5 mm per 1000 m and approximately 100 mm per 0.1 m, in particular between approximately 2 mm per 20 m and approximately 9 mm per 200 m.

An inner volume of the tubular flow path may be between approximately 1 ml and approximately 10.000 l, in particular between approximately 100 ml and approximately 100 l, further particular between approximately 250 ml and approximately 10 l. Larger volumes allow processing of larger amounts of the process fluid. However, the larger the volume, the lower the control of the processing due to increased occurrence of cross-flow currents within the tubular flow path.

In particular, larger volumes may allow a higher flow velocity. At a higher flow velocity, a lower residence time distribution may be obtained. While a higher flow velocity may be advantageous, a possible minimum residence time of the process fluid within the reactor module would need to be considered depending on design specifications.

The tubular flow path may comprise a Reynolds number of below 2300, in particular between 100 and 1000 at least in parts, preferably over its whole extent. At a Reynolds number of below 2300, substantially laminar flow occurs, which is advantageous for improved control of a reaction time of the process in the tubular flow path.

The tubular flow path may comprise a Dean number of between 54 and 1000. At a Dean number of 54 or above, perturbations and/or (Dean) vortices occur, which improves the mixing result.

The tubular flow path may be configured to guide the process fluid at a flow rate of between approximately 0,06 l/h (1 ml/min) and approximately 1000 l/h (16666 ml/min), in particular between approximately 1 l/h (16 ml/min) and 22 l/h (337 ml/min). The flow rate and the tubular flow path length substantially define the reaction time and/or residence time of the process fluid within the tubular flow path.

The tubular flow path may be configured to guide a process fluid having a viscosity of between approximately 0.2 mPas and approximately 10000 mPas, in particular between approximately 1 mPas and approximately 5 mPas.

The tube reactor module may further comprise one or more connecting sections, wherein at least one connecting section is positioned between sequential curve sections and fluidly connects said sequential curve sections with one another. Preferably, each connecting section is substantially straight.

The connecting sections may comprise a substantially constant cross-section, which may be substantially identical to the second cross-section at an end portion of the curve sections.

The connecting sections may comprise a connecting section length between approximately 1 mm and approximately 30 mm, in particular between approximately 5 mm and approximately 15 mm.

In particular, the connecting sections may be configured to allow turbulences in the process fluid caused by a change of flow direction to settle or at least reduce before reaching the sequential curve section. This is advantageous for obtaining less pressure drop and a smoother flow of the process fluid through the tubular flow path.

Nevertheless, said turbulences in the process fluid reduce residence time distribution. Accordingly, it is particularly advantageous that the connecting sections are configured so that the turbulences in the process fluid caused by the change of flow direction partially settle but do not entirely stop in order to obtain a low residence time distribution and a low pressure drop.

Also, the connecting sections may be configured to extend the tubular flow path length in order to obtain a larger volume of the tubular flow path and/or a longer residence time of the process fluid in the tubular flow path.

A ratio between a connecting section cross-section diameter and a connecting section length may be between approximately 0.5 mm and approximately 100 mm to between approximately 0.1 mm to approximately 1 m, in particular between approximately 3 mm to approximately 11 mm and approximately 9 mm to approximately 11 mm.

The tube reactor module may comprise a first formed sheet and a second formed sheet, wherein the first formed sheet and the second formed sheet respectively comprise a conduit structure embedded in substantially planar joining surface of the first and second formed sheets, wherein the conduit structures form at least a part of the tubular flow path in a state in which the first formed sheet and the second formed sheet are joined together at their respective joining surfaces.

A respective tube reactor module is advantageous because it allows a compact design and/or a low ratio of weight to volume of the tubular flow path. Also, a respective tube reactor module has low material usage for improved cost efficiency and reduced climate footprint and is lightweight for easy handling.

Preferably, each conduit structure of the first formed sheet and the second formed sheet includes and/or forms substantially 50% of the cross-sectional area of the tubular flow path and/or substantially 100% of the length of the tubular flow path. When joined together, the first formed sheet and the second formed sheet form the tubular flow path for guiding the process fluid.

The formed sheets may be manufactured using thermoforming and/or vacuum forming.

The first formed sheet and/or the second formed sheet may comprise or consist of plastic material, such as ABS (Acrylonitrile butadiene styrene) and/or other, in particular thermoformable, materials such as PETG or PET-G (Polyethylene terephthalate glycol) and/or PMMA (Polymethyl methacrylate) and/or UHMW (ultrahigh molecular weight polyethylene) and/or EVA (Ethylene-vinyl acetate) and/or HIPS (High impact polystyrene) and/or PP (Polypropylene) and/or PC (Polycarbonate) and/or LDPE (Low-Density Polyethylene) and/or HDPE (High-Density Polyethylene) and/or PVC (Polyvinyl Chloride).

However, the tube reactor module may be manufactured using other manufacturing methods, such as injection molding, additive manufacturing and/or shape cutting, and/or consist of and/or comprise materials, such as metal or other plastic materials or biodegradable materials.

Preferably, the inlet and/or the outlet comprise a fitting configured for substantially fluid tight coupling with a hose and/or a tube and/or another connector for receiving or dispensing the process fluid.

A further aspect of the invention relates to a tube reactor system comprising at least a first tube reactor module as described herein and a second tube reactor module as described herein, wherein the outlet of the first tube reactor module is fluidly connected to the inlet of the second tube reactor module.

The tube reactor system may further comprise a third tube reactor module as described herein, wherein the outlet of the second tube reactor module is fluidly connected to the inlet of the third tube reactor module.

The tube reactor system may further comprise a fourth tube reactor module as described herein, wherein the outlet of the third tube reactor module is fluidly connected to the inlet of the fourth tube reactor module.

The tube reactor system may further comprise a fifth tube reactor module as described herein, wherein the outlet of the fourth tube reactor module is fluidly connected to the inlet of the fifth tube reactor module.

The tube reactor system may comprise between 2 and 1000 tube reactor modules, in particular between 2 and 10 tube reactor modules.

Respective tube reactor systems are advantageous because multiple tube reactor modules may be fluidly coupled to one another so as to obtain a tube reactor system with a desired tubular flow path length. In particular, it allows manufacturing of multiple identical tube reactor modules, which reduces costs.

The combined tubular flow path of all tube reactor modules are fluidly connected so as to form a tubular flow path extending from the inlet of the first tube reactor module to the outlet of the last tube reactor module in series.

The outlet of a preceding tube reactor module may be directly coupled to the inlet of a sequential tube reactor module. Alternatively, a preceding tube reactor module may be indirectly coupled to the inlet of a sequential tube reactor module, for example by a fluid connector.

The first tube reactor module and the second tube reactor module may respectively comprise complementary alignment means for guiding an alignment of the first tube reactor module and the second tube reactor module side by side and/or parallel and/or in a stacked and/or overlapping configuration, wherein the alignment means preferably are configured to produce a form fit between the first tube reactor module and the second tube reactor module.

For example, the first tube reactor module may comprise an alignment means having or being a protuberance and/or a plug, wherein the second tube reactor module comprises an alignment means having or being a recess and/or a socket for receiving the alignment means of the first tube reactor module.

The same applies for further tube reactor modules of the tube reactor system.

The tube reactor modules may comprise a first formed sheet and a second formed sheet as described above. A tube reactor system comprising respective tube reactor modules is particularly advantageous because it allows a compact shape of the tube reactor system. In particular, the tube reactor system may comprise a rack for holding a plurality of tube reactor modules side by side and/or in a stacked configuration, wherein two or more tube reactor modules may be provided depending on the desired tubular flow path length. This obtains a flexible design of the tube reactor system.

A further aspect of the invention relates to use of a tube reactor module as described herein and/or a tube reactor system as described herein for dynamic, in particular continuous, viral inactivation in a process fluid.

In particular, the tube reactor module and/or the tube reactor system may be used for viral inactivation at a pH level < 7 or at a pH > 7 and/or with detergents and/or other chemicals.

Alternatively or in addition, the tube reactor module and/or the tube reactor system may be used for dynamic, in particular continuous, operation and/or processing, or batch processing, or a hybrid thereof with temporarily interrupted flow of the process fluid flow.

The tube reactor module and/or the tube reactor system may be used for residence times of a process fluid between a few seconds, in particular between 5 and 40 seconds, and several hours, in particular between 2 to 48 hours. As a particular example, the tube reactor module and/or the tube reactor system may be used for a residence time of approximately 30 to 35 minutes for viral inactivation in the process fluid.

A further aspect of the invention relates to an incubation device comprising at least one tube reactor module as described herein or a tube reactor system as described herein.

The present invention is further explained in detail by the following detailed description and the appended drawings, in which particular embodiments are illustrated by way of example, wherein the present invention is in no way limited by these particular embodiments.

### Brief description of the drawings

- Fig. 1: shows an exemplary tube reactor module;
- Fig. 2: shows an inlet and a part of the tubular flow path of an exemplary tube reactor module;
- Fig. 3: shows a schematic of an exemplary tubular flow path of a tube reactor module;
- Fig. 4: shows a schematic of another exemplary tubular flow path of a tube reactor module;
- Fig. 5: shows a part of the exemplary tubular flow path of Fig. 3;
- Fig. 6a: shows a cross-sectional view of the tube reactor module along line a-a in Fig. 5;
- Fig. 6b: shows a cross-sectional view of the tube reactor module along line b-b in Fig. 5;
- Fig. 7: shows an exemplary tube reactor system comprising a plurality of fluidly connected tube reactor modules;
- Fig. 8: shows an exemplary first formed sheet and an exemplary second formed sheet together forming a tube reactor module.

### Description of particular examples

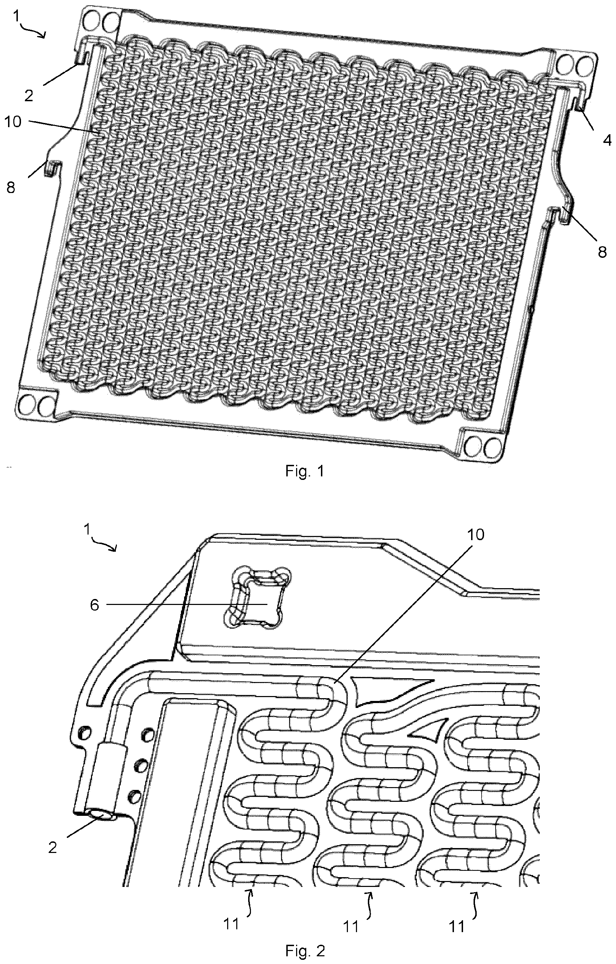

**Fig. 1** shows an exemplary tube reactor module 1 comprising a tubular flow path 10 comprising a serpentine pattern for guiding a process fluid from an inlet 2 to an outlet 4. The tubular flow path 10 of the exemplary tube reactor module 1 shown in Fig. 1 has a substantially planar configuration. The tubular flow path 10 is arranged within a flat plane and guides the process fluid in a meandering path across said plane.

In particular, the tubular flow path 10 comprises a repeating structure comprising plurality of serpentine turns. This allows for a substantially uniform flow characteristic of the process fluid over the whole extent of the tubular flow path 10.

The shown tube reactor module 1 corresponds to a particular advantageous example because it comprises an increased tubular flow path length in a compact shape with an effective mixing of the process fluid.

The tube reactor module 1 may comprise one or more flanges 8 and/or attachment portions for being held by a holder or rack, for example of an incubation device.

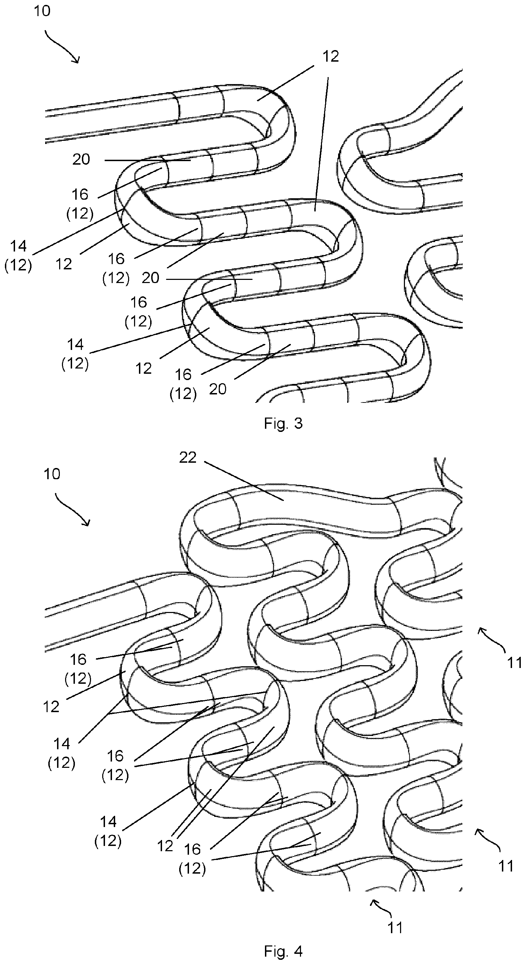

The tube reactor module 1 may comprise one or more of the features described with respect to other examples herein, in particular a tubular flow path as shown in Figs. 3 or 4.

**Fig. 2** shows an inlet 2 and a part of the tubular flow path 10 of an exemplary tube reactor module 1.

The tubular flow path 10 comprises a plurality of serpentine and/or meander sections 11, which are positioned adjacent to one another and fluidly connected to one another. The serpentine sections 11 include sequential turns bent in alternate directions.

Particular configurations of the tubular flow path 10 are shown in Figs. 3 and 4 and described further below herein.

At the area of the inlet 2, the tube reactor module 1 may comprise provisions for overmolding of a fitting for substantially fluid tight coupling of the inlet 2 with a hose and/or a tube and/or another connector for receiving or dispensing the process fluid.

The same or different provisions may be present at the are of the outlet 4 (not shown).

Alternatively, the inlet 2 and/or the outlet 4 may be configured for substantially fluid tight coupling with a hose and/or a tube and/or another connector and/or another tube reactor module 1.

The shown tube reactor module 1 further comprises an alignment means 6 for coupling with corresponding alignment means 6 of other tube reactor modules 1. In this example, the alignment means 6 comprises a recess and/or an indentation and/or socket which may be coupled with a complementary protuberance and/or pin and/or plug of another tube reactor module 1. Preferably, alignment means 6 of different tube reactor modules 1 produce a form-fit so as to prohibit or at least reduce relative movement between respective tube reactor modules 1 at least in one direction, preferably in two directions.

**Fig. 3** shows a schematic of an exemplary tubular flow path 10 of a tube reactor module 1. The tubular flow path 10 comprises a plurality of curve sections 12 so as to form a serpentine and/or meander configuration. Sequential curve sections 12 are bent in alternating directions. In the shown example, sequential curve sections 12 are bent in opposite directions.

The curve sections 12 are configured to change the flow direction of the process fluid by at least 90°. In the shown example, the curve sections 12 change the flow direction of the process fluid by approximately 180°. Thus, the flow direction of the process fluid is substantially parallel at or immediately before entering the end portions 16 of the corresponding curve section 12 and at or immediately after exiting the opposite end portions 16 of the corresponding curve section 12.

The shown configuration of the tubular flow path 10 with sequential curve sections 12 bent in opposite directions and changing the flow direction of the process fluid by approximately 180° allows a compact design of the tube reactor module 1 while providing effective mixing of the process liquid at a low residence time distribution of the process liquid.

The shown tubular flow path 10 comprises connecting sections 20, wherein at least one connecting section 20 is positioned between and fluidly, preferably directly, connects two sequential curve sections 12. The connecting sections 20 extend the tubular flow path length and may allow turbulences in the process fluid to settle or at least reduce before reaching the sequential curve section 12.

The connecting sections 20 are preferably substantially straight and/or oriented substantially parallel to one another.

The connecting sections 20 may comprise a connecting section length between approximately 1 mm and approximately 30 mm, in particular between approximately 5 mm and approximately 15 mm, for example approximately 11 mm.

The tubular flow path 10 may comprise differing cross-sections along its length. A cross-section is oriented substantially perpendicular to a (local) flow direction of the process fluid. In the particular example shown in Fig. 3, the shape of the cross-section of the curve sections 12, preferably gradually, changes from a substantially circular cross-section at a first end portion 16 to a substantially oval and/or elliptical cross-section at an apex 14 of the curve sections 12 to a substantially circular cross-section at a second end portion 16 of the curve sections 12.

Alternatively or in addition, the cross-sections along the tubular flow path 10 may differ with respect to the cross-section area and/or the size. A respective or similar change of cross-section in the curve sections 12 causes increased occurrence of turbulences including whirls and/or vortices in the process fluid through which improved mixing of the process fluid may be obtained.

The connecting sections 20 preferably comprise a substantially constant cross-section, wherein the cross-section is advantageously substantially identical to the cross-sections of end portions 16 of connected curve sections 12.

**Fig. 4** shows a schematic of another exemplary tubular flow path 10, which may comprise a similar configuration as the tubular flow path 10 shown in Fig. 3 with the difference that no section or only a section of marginal length with a substantially constant cross-section is provided between sequential curve sections 12. In other words: the cross-section of the tubular flow path 10 substantially continuously changes between apexes 14 of sequential curve sections 12.

The exemplary tubular flow path 10 shown in Fig. 4 allows for large ratio between number of curve sections 12 and tubular flow path length. For example, a respective configuration is advantageous for low residence time of the process fluid or short tubular flow path length yet effective mixing.

As shown in Fig. 4, the tubular flow path 10 comprises a linking section 22, which fluidly couples sequential curve sections 12 of different serpentine sections 11. The linking section preferably comprises a substantially constant cross-section and preferably fluidly couples sequential curve sections 12 directly and in or close to the shortest way possible.

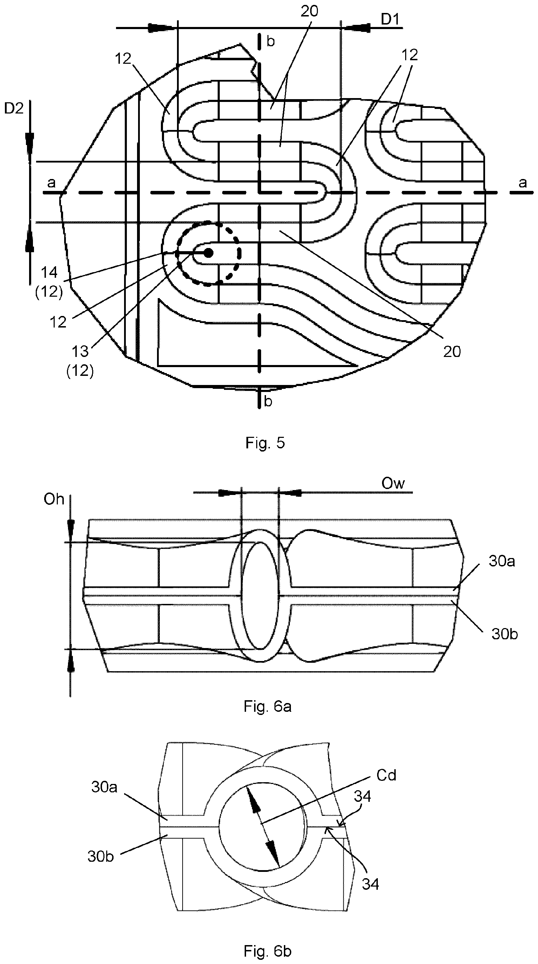

**Fig. 5** shows a part of the exemplary tubular flow path 10 of Fig. 3. Particular parameters of the tubular flow path 10 are illustrated. For example, the radius of the curve sections 12 is indicated as circle, which is defined by midpoints and/or central points along the tubular flow path 10 in the curve section 12. The center of said circle is positioned on an imaginary line connecting the inner wall and the outer wall at apex 14.

Also, a curve distance D1 is indicated, which may be measured between respective apexes 14 of sequential curve sections 12. The curve distance D1 may correspond to the distance between respective inner walls, mid points or outer walls at apexes 14 of sequential curve sections. The curve distance D1 may alternatively be measured in a direction substantially perpendicular to an imaginary line indicating the global flow direction of the process fluid over a section of the tubular flow path 10 of a corresponding serpentine section 11, i.e., parallel to line b-b. In this example, the measuring direction is parallel to line a-a shown in Fig. 5.

Also, a connecting section distance D2 is indicated, which may be measured between sequential connecting sections 20. The connecting section distance D2 may be measured in a direction substantially parallel to an imaginary line indicating the global flow direction of the process fluid over a section of the tubular flow path 10 of a corresponding serpentine section 11, i.e., parallel to line b-b shown in Fig. 5.

**Fig. 6a** shows a cross-sectional view of the tube reactor module 1 along line a-a in Fig. 5, specifically of a curved section 12 at its apex 14. The cross-section of the curved section 12 at its apex 14 comprises an oval and/or elliptical shape. In a particular advantageous example, the height of the cross-section Oh is approximately 10 mm and the width of the cross-section Ow is approximately 4 mm. Accordingly, a ratio between height Oh and width Ow is 2.5.

**Fig. 6b** shows a cross-sectional view of the tube reactor module along line b-b in Fig. 5, specifically of a connecting section 20. The cross-section of the connecting section 20 comprises a substantially circular shape. In a particular advantageous example, the diameter of the cross-section Cd is approximately 6,3 mm. Accordingly, a ratio between cross-sectional area of the apex 14 and cross-sectional area of the connecting section is 1.

The particular example corresponds to one of many possible tubular flow paths. Yet, said particular example offers a low and/or narrow residence time distribution, an acceptable pressure drop and an even velocity of the process liquid for a given range of process volume stream (l/h).

By variation of the configuration and/or design parameters of said particular example, other properties may be obtained, which may be desired and/or advantageous for particular processes and/or applications.

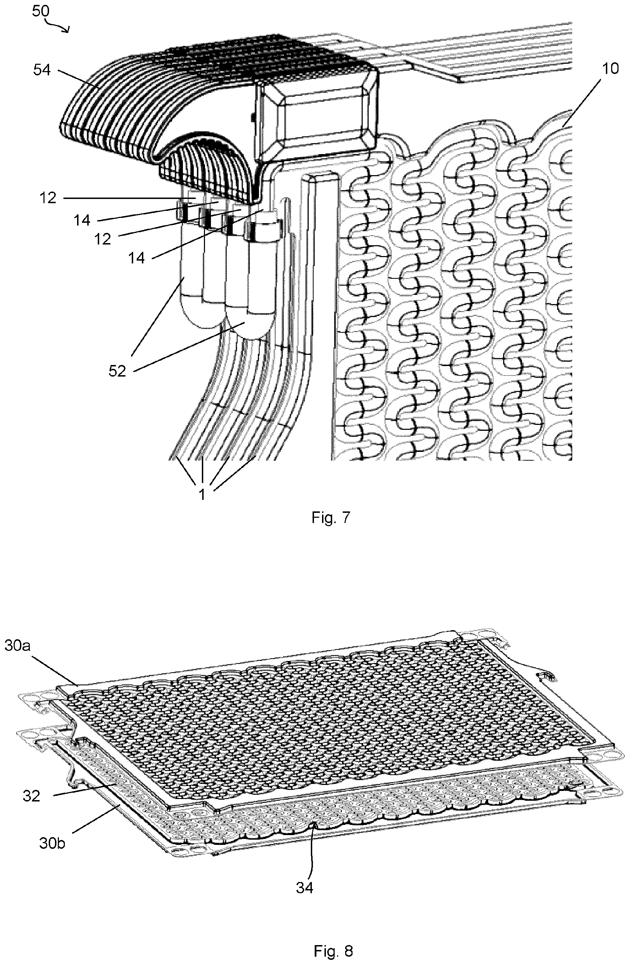

Furthermore, in Figs. 6a and 6b, a particular configuration of the tube reactor module 1 is illustrated. Specifically, the tube reactor module 1 comprises a first formed sheet 30a and a second formed sheet 30b joined at respective joining surface 34 thereby forming at least a part of the tubular flow path 10. Details in this respect are described herein, e.g. with respect to Fig. 8.

**Fig. 7** shows an exemplary tube reactor system 50 comprising a plurality of, specifically four, fluidly connected tube reactor modules 1. However, a tube reactor system 50 may comprise two or more, in particular 2, 3, 4, 5, 6, 7, 8, 9 or 10, tube reactor modules 1.

The tube reactor modules 1 advantageously have the identical configuration. This is advantageous because the plurality of reactor modules 1 may be positioned adjacent and/or in a stacked manner and/or side by side so as to be of compact size.

Optionally the plurality of tube reactor modules 1 may be retained and/or clamped by one or more retainer clips 54 so improved handling of the plurality of tube reactor modules 1 may obtained.

The plurality of tube reactor modules 1 are fluidly coupled so that their respective tubular flow paths 10 are combined so as to form a combined tubular flow path 10.

The outlet 14 of the first tube reactor module 1 is fluidly coupled to the inlet 12 of the second tube reactor module 1, preferably directly or by one or more fluid connectors 52.

According to a particular example, the tube reactor system 50 comprises four tube reactor modules 1 serially fluidly coupled to one another. Each tube reactor module 1 comprises a tubular flow path 10 of approximately 8.25 m. The combined tubular flow path 10 of the tube reactor system 50 is approximately 33 m. The volume of the combined tubular flow path 10 is approximately 980 ml. The average cross-section diameter of the tubular flow path 10 is approximately 6.2 mm.

A respective tube reactor system 50 comprises a residence time of the process fluid of between approximately 30 min to approximately 38 min, with a mean residence time of approximately 34 min, at a flow velocity of approximately 32 ml/min and a Reynolds number of approximately 130 and a Dean number of approximately 240.

As particularly apparent in Fig. 7, it is advantageous that the tubular flow path 10 is configured to guide the process fluid in either directions while achieving substantially identical mixing results. This allows that inlet 2 and outlet 4 may be switched so that, as illustrated, the outlet 4 of the first tube reactor module 1 situated at one end section of the first tube reactor module 1 is fluidly coupled to the inlet 2 of the second tube reactor module 1 situated at a corresponding and directly adjacent end section of the second tube reactor module 1. By this, a direct coupling between inlet 2 and outlet 4 may be obtained, which allows for a better mixing/tubular flow path length ratio and/or a more compact design and/or less material use. Alternatively, a fluid connector 52 fluidly coupling the tube reactor modules 1 may be short because it does not need to guide the process fluid to the opposing end section of the second tube reactor module 1.

Alternatively or in addition the tube reactor modules may comprise a substantially symmetrical form so that a tube reactor module 1 may be positioned in a rotated state so that the outlet 4 of the preceding tube reactor module 1 is directly adjacent to the inlet 2 of the sequential tube reactor module 1. In this configuration, the process liquid is guided through the tubular flow paths 10 of each tube reactor module 1 in the predetermined flow direction.

**Fig. 8** shows an exemplary first formed sheet 30a and an exemplary second formed sheet 30b together forming a tube reactor module 1 when joined at their respective joining surfaces 34.

A respective tube reactor module 1 is advantageous as it is low in material use, easy to manufacture and comprises a compact yet structurally robust design. Also, it offers a variety of possible layouts of the tubular flow path 10.

The first formed sheet 30a and the second formed sheet 30b each comprise a complementary conduit structure 32 at the joining surface 34. The conduit structure 32 forms a channel which is open towards the joining surface 34.

The conduit structure 32 forms a part, preferably approximately 50 %, of the cross-section of the tubular flow path 10 of the corresponding tube reactor module 1 with joined formed sheets 30a, 30b. For example, the sections of the conduit structure 32 of the first formed sheet 30a, which forms a circular connecting section 20 when joined to the second formed sheet 30b, comprises a substantially semicircle cross-section. Thus, when joined with the complementary section of the conduit structure 32 of the second formed sheet 30b, a connecting section 20 with a circular cross-section is formed.

### List of Reference Numerals

- 1: tube reactor module
- 2: inlet
- 4: outlet
- 6: alignment means
- 8: flange
- 10: tubular flow path
- 11: serpentine sections
- 12: curve section
- 13: radius of curve section
- 14: apex
- 16: end portion
- 20: connecting section
- 22: linking section
- 30a: first formed sheet
- 30b: second formed sheet
- 32: conduit structure
- 34: joining surface
- 50: tube reactor system
- 52: fluid connector
- 54: retainer clip
- D1: curve section distance
- D2: connecting section distance
- Ow: width of oval/elliptical cross-section at apex of curve section
- Oh: height of oval/elliptical cross-section at apex of curve section
- Cd: diameter of circular cross-section of connecting section

## Claims

1. Tube reactor module (1) for controlling a reaction time of a process fluid in dynamic operation, in particular during viral inactivation in the process fluid, comprising:
an inlet (2) for receiving the process fluid;
an outlet (4) for discharging the process fluid;
a tubular flow path (10) comprising a serpentine pattern for guiding the process fluid from the inlet (2) to the outlet (4), the tubular flow path comprising a sequence of fluidly connected alternatingly bent curve sections (12), wherein each curve section (12) is shaped such that a flow direction of the process fluid changes by at least approximately 90°, in particular by between approximately 135° and 225°, and
wherein the tubular flow path (10) comprises a first cross-section perpendicular to the flow direction of the process fluid at respective apexes (14) of the curve sections (12) and a second cross-section perpendicular to the flow direction of the process fluid at respective end portions (16) of the curve sections (12), wherein the first cross-section is different from the second cross-section.

2. Tube reactor module (1) of claim 1, wherein the shape of the first cross-section and the second cross-section of the tubular flow path (10) differs and, optionally, the first cross-section is substantially oval and/or elliptical and wherein the second cross-section is substantially circular.

3. Tube reactor module (1) of claim 1 or 2, wherein the tubular flow path (10) comprises 10 to 500, in particular 30 to 50, curve sections (12) per meter of a tubular flow path length.

4. Tube reactor module (1) of any one of the preceding claims, wherein a radius of the curve sections (12) is between approximately 2 mm and approximately 50 mm, in particular between approximately 5 mm and approximately 20 mm.

5. Tube reactor module (1) of any one of the preceding claims, wherein the first cross-section and/or the second cross-section comprises a cross-sectional area of between approximately 1 mm² and approximately 1000 mm², in particular between approximately 4 mm² and approximately 200 mm².

6. Tube reactor module (1) of any one of the preceding claims, wherein a curve distance measured between respective apexes (14) of sequential curve sections (12) is between approximately 5 mm and approximately 100 mm, in particular between approximately 10 mm and approximately 30 mm.

7. Tube reactor module (1) of any one of the preceding claims, wherein the tubular flow path (10) comprises between 100 and 4000 curve sections (12) per 0.5 ml volume, in particular between 500 and 1500 curve sections (12) per 0.5 ml volume.

8. Tube reactor module (1) of any one of the preceding claims, wherein a ratio of a cross-section diameter of the first cross-section and/or the second cross-section and a tubular flow path length is between approximately 2 mm per 20 m and approximately 9 mm per 200 m.

9. Tube reactor module (1) of any one of the preceding claims, wherein an inner volume of the tubular flow path (10) is between approximately 100 ml and approximately 1000 l, in particular between approximately 250 ml and approximately 10 l.

10. Tube reactor module (1) of any one of the preceding claims, further comprising one or more connecting sections (20), wherein one connecting section (20) is positioned between sequential curve sections (12) and fluidly connects said sequential curve sections (12) with one another,
wherein each connecting section (20) is substantially straight and:
comprises a substantially constant cross-section, which is substantially identical to the second cross-section of the curve sections (12); and/or
comprises a connecting section length between approximately 1 mm to approximately 30 mm, in particular between approximately 5 mm and approximately 15 mm.

11. Tube reactor module (1) of any one of the preceding claims, comprising a first formed sheet (301) and a second formed sheet (30b), wherein the first formed sheet (30a) and the second formed sheet (30b) respectively comprise a conduit structure (32) embedded in a substantially planar joining surface (34) of the first and second formed sheets (30a, 30b), wherein the conduit structures (32) form at least a part of the tubular flow path (10) in a state in which the first formed sheet (30a) and the second formed sheet (30b) are joined together at their respective joining surfaces (34).

12. Tube reactor system (50) comprising at least a first tube reactor module (1) according to any one of the preceding claims and a second tube reactor module (1) according to any one of the preceding claims, wherein the outlet (4) of first tube reactor module (1) is fluidly connected to the inlet (2) of the second tube reactor module (1).

13. Tube reactor system (50) of claim 12, wherein the first tube reactor module and the second tube reactor module (1) respectively comprise corresponding alignment means (6) for guiding an alignment of the first tube reactor module (1) and the second tube reactor module (1) side by side, wherein the alignment means (6) are configured to produce a form fit between the first tube reactor module (1) and the second tube reactor module (1).

14. Use of the tube reactor module (1) according to any one of claims 1 to 11 for dynamic viral inactivation in a process fluid.

15. Incubation device comprising at least one tube reactor module (1) according to any one of claims 1 to 11 or a tube reactor system (50) according to claim 12 or 13.
